# EUROPEAN PATENT SPECIFICATION

(11) **EP 1 977 696 B1**
(45) Date of publication and mention of the grant of the patent: **13.01.2010**
(21) Application number: 08003025.7
(22) Date of filing: 19.02.2008
(51) Int. Cl.: A61B 17/00, A61B 17/32, A61B 19/00

(54) **Dissection apparatus**
Dissektionsvorrichtung
Appareil de dissection

(30) Priority: 06.04.2007 US 697502
(43) Date of publication of application: 08.10.2008
(73) Proprietor: Olympus Medical Systems Corporation, Tokyo 151-0072 (JP); Terumo Cardiovascular Systems Corporation, Ann Arbor MI 48103-9300 (US)
(72) Inventor: Ken, Yamatani c/o Olympus Intel. Prop. Services Co., Ltd., Hachioji-shi Tokyo 192-8512 (JP)
(74) Representative: von Hellfeld, Axel

(56) References cited:
- US-A- 6 019 720
- US-A1- 2005 159 764

## Description

The present invention relates to a dissection apparatus of a blood vessel from a living tissue.

Jpn. Pat. Appln. KOKAI Publication No. 9-75354 discloses a dissection apparatus to dissect a blood vessel from a blood vessel connective tissue in order to sample a subcutaneous blood vessel such as a saphenous vein of a lower limb under endoscopic observation. In the dissection apparatus, a distal end portion for dissection is disposed at the distal end of a cylindrical main body portion. The distal end portion includes a tapered conical shape as a whole so it can be easily inserted between the blood vessel connective tissue and the blood vessel, and its distal end forms a locally blunt shape so it will not apply any unwanted force to the blood vessel.

In the dissection apparatus of Jpn. Pat. Appln. KOKAI Publication No. 9-75354, as the distal end portion includes a conical shape, when dissecting a blood vessel, it is difficult to move the distal end portion of the apparatus along the blood vessel and dissection is performed by only moving the distal end portion forward. Therefore, the dissecting operation becomes cumbersome.

US 6 019 720 discloses a dissection apparatus according to the preamble of claim 1.

It is an object of the present invention to provide a dissection apparatus capable of performing dissection of a blood vessel easily and reliably.

In an aspect of the present invention, a dissection apparatus is characterized by including: a main body portion which extends from a proximal end side to a distal end side; and a dissecting portion which is provided to a distal end of the main body portion and adapted to dissect a blood vessel from a living tissue, the dissecting portion including a flat portion which is to be pressed against the blood vessel.

In the dissection apparatus, while pressing the flat portion against the blood vessel, the dissecting portion is moved forward or pivoted along the blood vessel to dissect the blood vessel from the living tissue. Therefore, in the dissecting operation, the dissecting portion rarely disengages from the blood vessel and the blood vessel can also be dissected by the pivotal motion of the dissecting portion in addition to the forward movement. This enables to perform dissection of the blood vessel easily and reliably.

In a preferred aspect of the present invention, the dissection apparatus is characterized in that the dissecting portion includes a diameter decreasing portion a diameter of which decreases from the proximal end side to the distal end side, and the flat portion is provided to the diameter decreasing portion.

In the dissection apparatus, as the diameter decreasing portion, which is inserted between the living tissue and the blood vessel, is provided with the flat portion, during insertion, the dissecting portion rarely disengages from the blood vessel and by pivoting the diameter decreasing portion inserted between the living tissue and the blood vessel, the blood vessel can be dissected easily.

In a preferred aspect of the present invention, the dissection apparatus is characterized in that the flat portion extends from a distal end of the dissecting portion to the proximal end side thereof.

In the dissection apparatus, as the flat portion extends from the distal end of the dissecting portion, the distal end of the dissecting portion rarely disengages from the blood vessel. This sufficiently prevents the entire dissecting portion from disengaging from the blood vessel.

In a preferred aspect of the present invention, the dissection apparatus is characterized in that the dissecting portion includes an air supply hole to supply gas from inside of the dissecting portion to outside thereof.

In the dissection apparatus, supply of air from the air supply hole enlarges an inner cavity to facilitate observation of the interior of the inner cavity with the endoscope and dissecting operation itself. This enables to perform dissection of the blood vessel sufficiently easily and reliably.

In a preferred aspect of the present invention, the dissection apparatus is characterized in that the dissection apparatus is used together with an endoscope, and the air supply hole is adapted to be arranged within a field of view of the endoscope.

In the dissection apparatus, the air supply state from the air supply hole can be observed with the endoscope. This enables accurate understanding of the air supply state.

In a preferred aspect of the present invention, the dissection apparatus is characterized in that the dissecting portion includes an air supply hole which is formed in the diameter decreasing portion to supply gas from inside of the dissecting portion to outside thereof.

In the dissection apparatus, the diameter decreasing portion, which is inserted between the living tissue and the blood vessel, is provided with the air supply hole. This enables to enlarge in particular the portion of an inner cavity, which tend to be clogged and affect observation and dissecting operation.

In a preferred aspect of the present invention, the dissection apparatus is characterized in that the air supply hole is arranged on a side of the flat portion in the dissecting portion.

In the dissection apparatus, the air supply hole is arranged, of the dissecting portion, neither on the flat portion nor on the side that opposes the flat portion that tend to be covered by the living tissue or the blood vessel, but on the side of the flat portion. This prevents the air supply hole from being clogged by the living tissue or blood vessel to interfere with air supply.

In a preferred aspect of the present invention, the dissection apparatus is characterized in that the air supply hole is arranged to oppose the flat portion in the dissecting portion.

In the dissection apparatus, as the air supply hole is arranged to oppose the flat portion, pressure generated by air supply from the air supply hole adapted to dissect the blood vessel from the living tissue. This enables promotion of dissection of the blood vessel by air supply.

In another aspect of the present invention, a dissection apparatus is characterized by including: a main body portion which extends from a proximal end side to a distal end side; and, a dissecting portion which is provided to a distal end of the main body portion and adapted to dissect a blood vessel from a living tissue, the dissecting portion including a transparent portion to observe an outside of the dissecting portion from inside thereof by an endoscope, and the transparent portion including a mark which is observable by the endoscope.

In the dissection apparatus, the relative positional relationship between the blood vessel and the dissecting portion can be understood from the relative positional relationship between the blood vessel and the mark in the field of view of the endoscope. This enables to perform dissection of the blood vessel easily and reliably.

In a preferred aspect of the present invention, the dissection apparatus is characterized in that the mark is arranged at a center of a field of view of the endoscope.

In the dissection apparatus, as the mark is arranged at the center of the field of view of the endoscope and usually, a dissection target portion to be dissected by the dissecting portion is arranged at the center of the field of view of the endoscope, the relative positional relationship between the dissection target portion and dissecting portion can be understood easily.

In a preferred aspect of the present invention, the dissection apparatus is characterized in that the mark is linear.

In the dissection apparatus, as the mark is linear and extends in a direction, the direction and the posture of the dissecting portion can be understood easily.

In a preferred aspect of the present invention, the dissection apparatus is characterized in that the mark is formed of an acute-angled shape of the transparent portion.

In the dissection apparatus, as the acute-angled shape to be inserted in a living tissue forms the mark, the relative positional relationship between the blood vessel and the acute-angled shape can be understood. This enables to perform dissection of the blood vessel sufficiently easily and reliably.

The invention can be more fully understood from the following detailed description when taken in conjunction with the accompanying drawings, in which:
FIG. 1 is a view showing a dissection system according to the first embodiment of the present invention;
FIG. 2A is a perspective view showing a dissecting portion according to the first embodiment of the present invention;
FIG. 2B is a top view showing the dissecting portion according to the first embodiment of the present invention;
FIG. 2C is a side view showing the dissecting portion according to the first embodiment of the present invention;
FIG. 3A is a top view showing a dissecting portion according to a modification of the first embodiment of the present invention;
FIG. 3B is a side view showing the dissecting portion according to the modification of the first embodiment of the present invention;
FIG. 4A is a schematic view showing a saphenous vein of a lower limb which is to be dissected by a dissection method according to the first embodiment of the present invention;
FIG. 4B is a schematic view showing a cutaneous vein of an upper limb which is to be dissected by the dissection method according to the first embodiment of the present invention;
FIG. 5 is a schematic view to explain the dissection method according to the first embodiment of the present invention;
FIG. 6A is a view showing an endoscopic image in a step of dissecting a main duct in the dissection method according to the first embodiment of the present invention;
FIG. 6B is a view showing an endoscopic image in a stage before a step of dissecting a branch in the dissection method according to the first embodiment of the present invention;
FIG. 6C is a view showing an endoscopic image in a step of dissecting the branch in the dissection method according to the first embodiment of the present invention;
FIG. 6D is a view showing an endoscopic image in a step of dissecting from an undissected tissue in the dissection method according to the first embodiment of the present invention;
FIG. 7A is a perspective view showing a dissecting portion according to the second embodiment of the present invention;
FIG. 7B is a top view showing the dissecting portion according to the second embodiment of the present invention;
FIG. 7C is a side view showing the dissecting portion according to the second embodiment of the present invention;
FIG. 8A is a top view showing a dissecting portion according to a modification of the second embodiment of the present invention;
FIG. 8B is a side view showing the dissecting portion according to the modification of the second embodiment of the present invention;
FIG. 9 is a view showing an endoscopic image in a dissection method according to the second embodiment of the present invention;
FIG. 10A is a perspective view showing a dissecting portion according to a third embodiment of the present invention;
FIG. 10B is a top view showing the dissecting portion according to the third embodiment of the present invention;
FIG. 10C is a side view showing the dissecting portion according to the third embodiment of the present invention;
FIG. 11 is a top view showing a dissecting portion according to the fourth embodiment of the present invention;
FIG. 12A is a perspective view showing a dissecting portion according to a fifth embodiment of the present invention;
FIG. 12B is a top view showing the dissecting portion according to the fifth embodiment of the present invention;
FIG. 12C is a side view showing the dissecting portion according to the fifth embodiment of the present invention; and
FIG. 13 is a schematic view to explain a dissection method according to the fifth embodiment of the present invention.

The respective embodiments of the present invention will be described hereinafter with reference to the accompanying drawing.

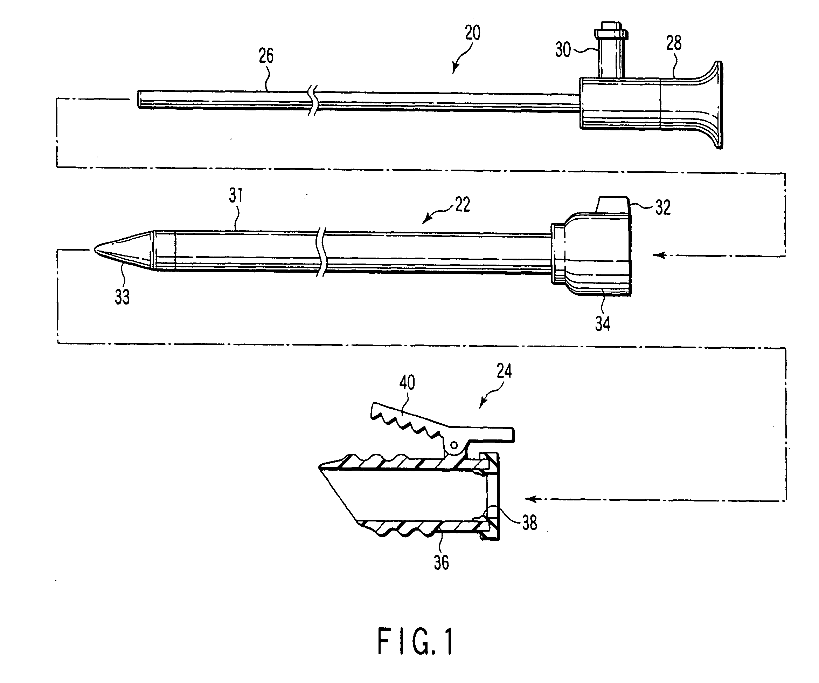

FIGS. 1 to 6D show the first embodiment of the present invention and its modification.

Referring to FIG. 1, a dissection system according to the embodiment includes an endoscope 20 to perform observation in a body, a dissection apparatus 22 to dissect a blood vessel in the body, and a trocar 24 to insert the endoscope 20 and dissection apparatus 22 into the body.

The endoscope 20 is a rigid endoscope and includes an elongated rod-like inserting portion 26. The proximal end of the inserting portion 26 connects to an eyepiece 28 to observe an endoscopic image. A lightguide connecter 30 projects from the eyepiece 28. The lightguide connecter 30 connects to a lightguide cable to supply illumination light to the endoscope 20.

The dissection apparatus 22 includes a tubular main body portion 31 where the endoscope 20 is to be inserted to be movable forward/backward. A connecting portion 34 to connect to the eyepiece 28 of the endoscope 20 is disposed on the proximal end of the . main body portion 31. More specifically, the connecting portion 34 includes a notch 32 where the lightguide connecter 30 of the endoscope 20 is to be inserted and engaged. A dissecting portion 33 to dissect a blood vessel is disposed at the distal end of the main body portion 31. The dissecting portion 33 will be described later in detail.

The trocar 24 includes a guide pipe 36 to guide the dissection apparatus 22 into the body. A seal 38 is disposed on the inner surface of the proximal end of the guide pipe 36, The seal 38 allows the dissection apparatus 22 to be inserted in the guide pipe 36 airtightly and to be movable forward/backward. The outer surface of the guide pipe 36 includes a projection to engage with the living tissue. The trocar 24 also includes a holding portion 40 to hold the guide pipe 36 onto the living tissue.

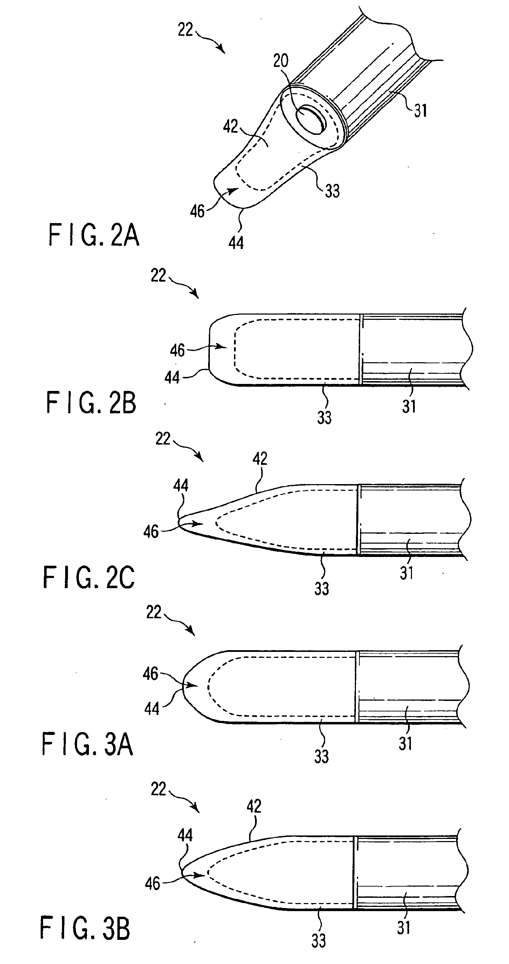

The dissecting portion 33 will be described in detail with reference to FIGS. 2A to 2C.

The dissecting portion 33 according to the embodiment forms a wide shape extending from the proximal end side to the distal end side while keeping almost the same width when seen in one direction perpendicular to the longitudinal direction of the main body portion 31 (to be referred to as the vertical direction hereinafter), and a tapered shape when seen in a direction perpendicular to the longitudinal direction of the main body portion 31 and one direction described above (to be referred to as the right-and-left direction hereinafter). In other words, the entire dissecting portion 33 forms a diameter decreasing portion with a diameter that decreases from the proximal end side to the distal end side. The upper surface of the dissecting portion 33 includes a rectangular flat portion 42 which extends from the distal end toward the proximal end side of the dissecting portion 33 while keeping almost the same width. The flat portion 42 is to be pressed against a blood vessel.

The dissecting portion 33 is formed of a hollow transparent member including an almost constant thickness. The entire dissecting portion 33 forms a transparent portion. By the endoscope 20 which is inserted in the main body portion 31 and projects from the distal end of the main body portion 31, observation of the outside from the inside of the dissecting portion 33 is enabled. At the distal end of the dissecting portion 33, an acute-angled shape 44 which forms an acute angle when seen in the right-and-left direction extends in the direction of width when seen in the vertical direction. The distal end of the acute-angled shape 44 locally forms a blunt shape. The inner surface of the dissecting portion 33 includes a shape that corresponds to its outer surface. Bend line portions on the outer surface and the inner surface of the acute-angled shape 44 form a linear mark 46 which can be observed by the endoscope 20. The mark 46 is to be arranged at almost the center of the field of view of the endoscope 20.

FIGS. 3A and 3B show a modification of the embodiment.

In the dissecting portion 33 according to this modification, when seen in the vertical direction, at its distal end side, the width decreases from the proximal end side toward the distal end side almost axi-symmetrically about the center axis. Note that when seen in the vertical direction, the distal end side of the dissecting portion 33 does not converge to a vertex. At the distal end of the dissecting portion 33, the acute-angled shape 44 similar to that of the first embodiment extends in the direction of width. The dissecting portion 33 of this modification also forms the linear mark 46.

A method for using the dissection apparatus 22 according to the embodiment will be described.

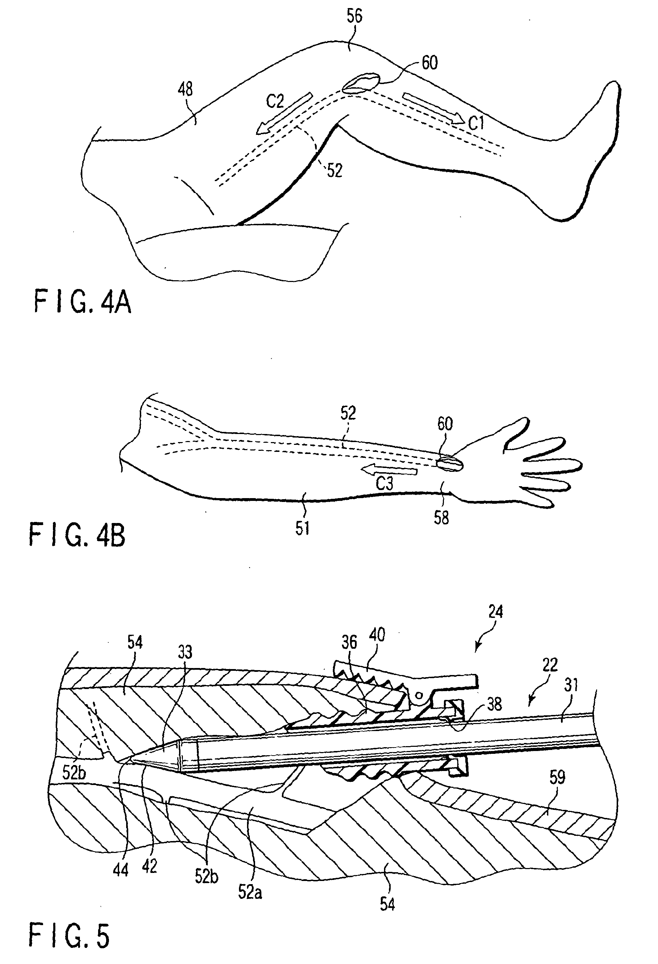

Referring to FIGS. 4A, 4B, and 5, an operation of sampling a blood vessel 52 such as a saphenous vein of a lower limb 48 or a cutaneous vein of an upper limb 51 is performed to use the sampled blood vessel 52 in coronary artery bypass surgery. When sampling the blood vessel 52, the blood vessel 52 must be dissected from a blood vessel connective tissue 54. In the following description, a dissection method of the blood vessel 52 will be described in detail in respective steps.

### Preparation Step

Referring to FIGS. 4A, 4B, and 5, in a knee 56 or wrist 58, a skin 59 is incised immediately above the blood vessel 52 to be sampled, to form an incision 60. The guide pipe 36 of the trocar 24 is inserted in the incision 60 and held by the holding portion 40 with respect to the incision 60. The endoscope 20 is inserted in the dissection apparatus 22. The lightguide connecter 30 of the endoscope 20 is inserted in and engaged with the notch 32 of the dissection apparatus 22, to lock the dissection apparatus 22 and endoscope 20 with each other. The distal end of the endoscope 20 is caused to project from the distal end of the main body portion 31 of the dissection apparatus 22 and arranged within the dissecting portion 33. Subsequently, the endoscope 20 and dissection apparatus 22 are inserted into the body through the trocar 24. During this insertion, due to the seal 38 of the trocar 24, the dissection apparatus 22 is inserted in the guide pipe 36 airtightly and to be movable forward/backward.

### Step of Dissecting Main Duct

As indicated by arrows C1, C2, and C3 in FIGS. 4A and 4B, the dissection apparatus 22 inserted in the body dissects a main duct 52a of the blood vessel 52 from the blood vessel connective tissue 54.

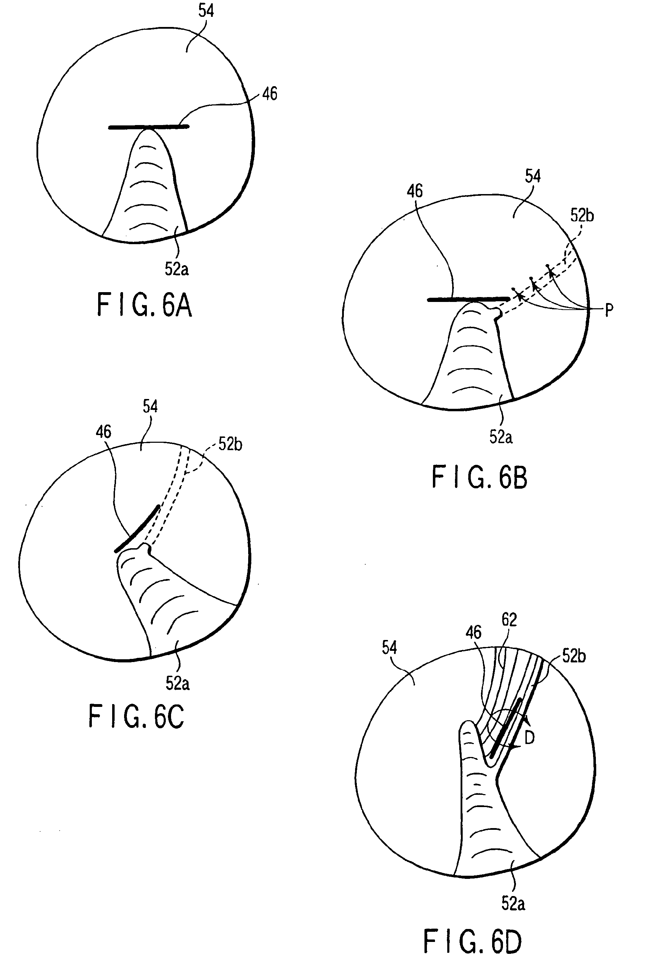

More specifically, as shown in FIG. 6A, in the observation image of the endoscope 20, the linear mark 46 is arranged at almost the center of the field of view of the endoscope 20. The mark 46 indicates the acute-angled shape 44 of the dissecting portion 33. The relative positional relationship between the acute-angled shape 44 and main duct 52a can be understood from the relative positional relationship between the mark 46 and main duct 52a. By manipulating the dissection apparatus 22, the dissecting portion 33 is arranged such that its flat portion 42 faces the main duct 52a and the main duct 52a extends from the peripheral portion to the central portion in the observation image of the endoscope 20. While keeping the mark 46 in constant contact with the outer surface of the main duct 52a in the observation image of the endoscope 20, the dissection apparatus 22 is pushed in. Consequently, with the flat portion 42 being pressed against the main duct 52a, the dissecting portion 33 is moved forward along the main duct 52a, and the acute-angled shape 44 at the distal end of the dissecting portion 33 is inserted between the blood vessel connective tissue 54 and the main duct 52a to dissect the main duct 52a from the blood vessel connective tissue 54.

### Step of Dissecting Branch

Subsequently, a branch 52b branching from the main duct 52a is dissected from the blood vessel connective tissue 54.

More specifically, first, as shown in FIG. 6B, the branch 52b is detected in the observation image of the endoscope 20.

If the dissecting portion includes a conical shape, its distal end is moved across the branch 52b and inserted between the blood vessel connective tissue 54 and the branch 52b. By repeating this insertion at a large number of positions P along the running direction of the branch 52b, the branch 52b is dissected.

In contrast to this, with the dissection apparatus 22 according to the embodiment, as shown in FIG. 6C, the dissection apparatus 22 is manipulated to arrange the dissecting portion 33 such that the flat portion 42 of the dissecting portion 33 faces the branch 52b, and that the mark 46 is located at the boundary portion of the blood vessel connective tissue 54 and the branch 52b to be almost parallel to the running direction of the branch 52b in the observation image of the endoscope 20. The dissection apparatus 22 is then pushed in. Consequently, the acute-angled shape 44 at the distal end of the dissecting portion 33 is inserted between the blood vessel connective tissue 54 and the branch 52b. With the flat portion 42 being pressed against the branch 52b, the dissecting portion 33 is moved forward across the branch 52b, to dissect the branch 52b from the blood vessel connective tissue 54.

### Step of Dissecting from Undissected Tissue

As shown in FIG. 6D, an undissected tissue 62 such as a funicular tissue may be left near the branch 52b. In this case, in the same manner as in the step of dissecting the branch, the dissection apparatus 22 is manipulated to arrange the dissecting portion 33 such that its flat portion 42 faces the branch 52b, and that the mark 46 is located at a boundary portion of the branch 52b and the undissected tissue 62 to be almost parallel to the running direction of the branch 52b in the observation image of the endoscope 20. The dissection apparatus 22 is then pushed in. Consequently, the acute-angled shape 44 at the distal end of the dissecting portion 33 is inserted between the undissected tissue 62 and branch 52b. With the dissecting portion 33 being inserted between the undissected tissue 62 and branch 52b, the dissection apparatus 22 is rotated about its central axis. The dissecting portion 33 is pivoted while pressing the flat portion 42 against the branch 52b, as indicated by an arrow D in FIG. 6D, to dissect the branch 52b from the undissected tissue 62.

Where necessary, in the step of dissecting the main duct, with the dissecting portion 33 being inserted between the blood vessel connective tissue 54 and the main duct 52a, the dissection apparatus 22 may be rotated about its central axis and dissecting portion 33 may be pivoted with the pressing portion of the main duct 52a and the flat portion 42 as a fulcrum, to dissect the main duct 52a from the blood vessel connective tissue 54.

Similarly, in the step of dissecting the branch, with the dissecting portion 33 being inserted between the blood vessel connective tissue 54 and the branch 52b, the dissection apparatus 22 may be rotated about its central axis and the dissecting portion 33 may be pivoted while pressing the flat portion 42 against the branch 52b, to dissect the branch 52b from the blood vessel connective tissue 54.

Therefore, the dissection apparatus 22 according to the embodiment includes the following effects.

In the dissection apparatus 22 according to the embodiment, in the dissecting operation, the dissecting portion 33 rarely disengages from the main duct 52a or branch 52b and the main duct 52a and branch 52b can be dissected by the pivotal motion of the dissecting portion 33 in addition to the forward movement. This enables to perform dissection of the main duct 52a and branch 52b easily and reliably. In particular, as the dissecting portion 33 that forms the diameter decreasing portion is provided with the flat portion 42, when inserting the dissecting portion 33 between the blood vessel connective tissue 54 and the main duct 52a or branch 52b, the dissecting portion 33 rarely disengages from the main duct 52a or branch 52b and by pivoting the dissecting portion 33 inserted between the blood vessel connective tissue 54 and the main duct 52a or branch 52b, the main duct 52a or branch 52b can be dissected easily. As the flat portion 42 extends from the distal end of the dissecting portion 33, the distal end of the dissecting portion 33 rarely disengages from the main duct 52a or branch 52b. This sufficiently prevents the entire dissecting portion 33 from disengaging from the main duct 52a or blood vessel 52.

The relative positional relationship between the main duct 52a or branch 52b and the dissecting portion 33 can be understood from the relative positional relationship between the main duct 52a or branch 52b and the mark 46 in the field of view of the endoscope 20. In particular, the acute-angled shape 44 to be inserted in the boundary region of the blood vessel connective tissue 54 and the main duct 52a or branch 52b forms the mark 46, the mark 46 is arranged at the center of the field of view of the endoscope 20, and the boundary region as the dissection target portion is also arranged at the center of the field of view of the endoscope 20. Thus, the relative positional relationship between the boundary region as the dissection target portion and the acute-angled shape 44 can be understood easily. As the mark 46 is linear and extends in a direction, the direction and posture of the dissecting portion 33 can be understood easily. Hence, dissection of the main duct 52a or branch 52b can be performed easily and reliably.

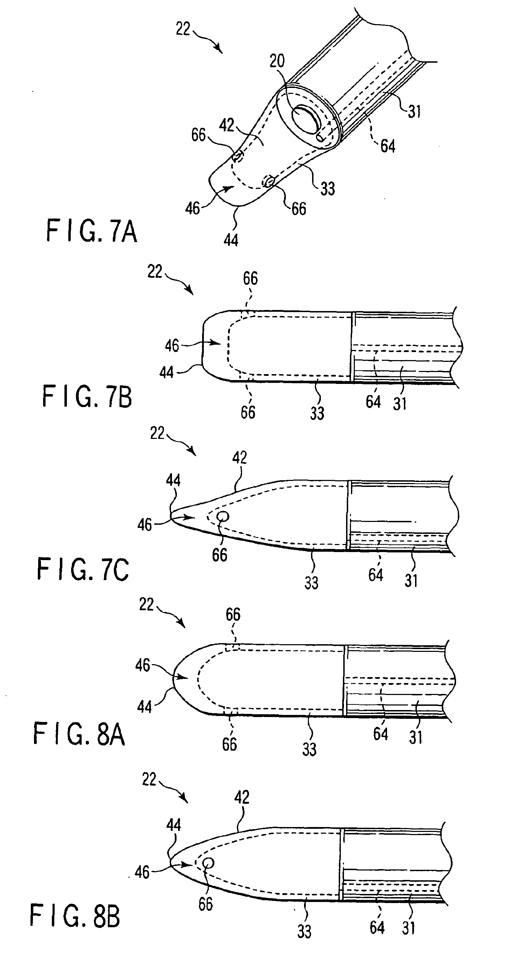

FIGS. 7A to 9 show the second embodiment of the present invention and its modification.

In a dissection apparatus 22 according to the embodiment, an air supply path 64 extends in its main body portion 31. The proximal end of the air supply path 64 communicates with an air supply connecter projecting from the proximal end of the main body portion 31. The distal end of the air supply path 64 opens to the interior of the dissecting portion 33 at the distal end of the main body portion 31. At the distal end of the main body portion 31, the endoscope 20 is airtightly projected/retracted. The distal end of the dissecting portion 33 includes air supply holes 66 through which the inside and outside of the dissecting portion 33 communicate with each other. The air supply holes 66 are arranged in the dissecting portion 33, which forms the diameter decreasing portion, on the two sides of the flat portion 42 when seen in the vertical direction, to be held within the field of view of the endoscope 20, and open to the right-and-left direction.

FIGS. 8A and 8B show the modification of the embodiment.

The dissecting portion 33 of the embodiment includes the same shape as that of the dissecting portion 33 of the modification of the first embodiment, and the air supply hole 66 similar to each of those of the second embodiment.

A method for using the dissection apparatus 22 according to the embodiment will be described.

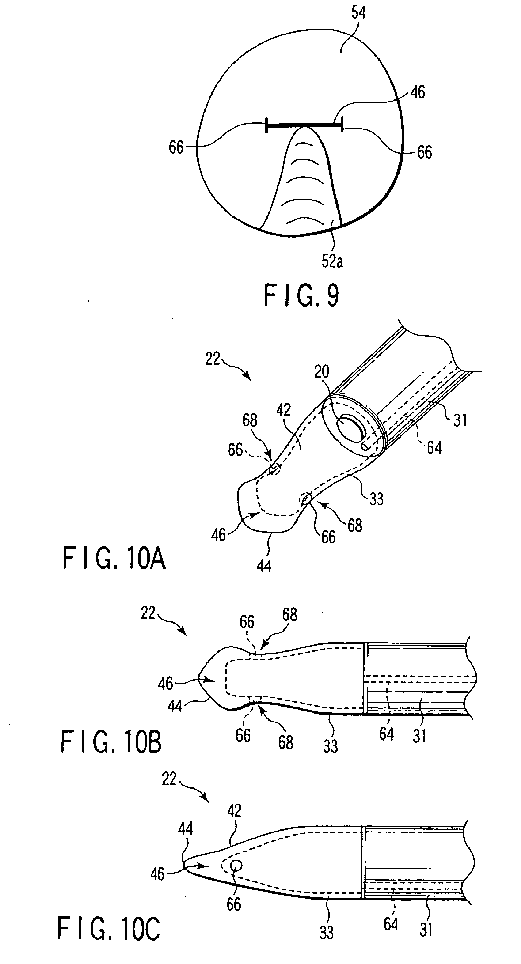

The dissecting operation of the blood vessel 52 by the dissection apparatus 22 according to the embodiment is the same as that by the dissection apparatus 22 according to the first embodiment. Note that air is supplied from the air supply holes 66 through the air supply connecter, air supply path 64, and the interior of dissecting portion 33 to enlarge the inner cavity where the dissecting operation is to be performed. By observing the states of the blood vessel connective tissue 54 and the main duct 52a or the branch 52b near the air supply hole 66 by the endoscope 20, as shown in FIG. 9, the air supply state from the air supply holes 66 can be understood.

Therefore, the dissection apparatus 22 according to the embodiment includes the following effects.

In the dissection apparatus 22 according to the embodiment, air supplied from the air supply holes 66 enlarges the inner cavity. In particular, the air supply holes 66 are formed at the dissecting portion 33 which forms the diameter decreasing portion to be inserted between the blood vessel connective tissue 54 and the main duct 52a or branch 52b. This enables to enlarge the portion of the inner cavity, which tend to be clogged and affect observation and dissecting operation. The air supply holes 66 are arranged, of the dissecting portion 33, neither on the flat portion 42 nor on the side that opposes the flat portion 42 that can be easily covered by the main duct 52a, the branch 52b, or the blood vessel connective tissue 54, but on the two sides of the flat portion 42. This prevents the air supply holes 66 from being clogged to interfere with air supply. Hence, observation of the interior of the inner cavity by the endoscope 20 and the dissecting operation itself becomes easy, so dissection of the main duct 52a or branch 52b can be performed sufficiently easily and reliably.

FIGS. 10A to 10C show the third embodiment of the present invention.

According to the embodiment, the width of the dissecting portion 33 decreases once, and then increases and decreases again from the proximal end side toward the distal end side when seen in the vertical direction, so the distal end portion of the dissecting portion 33 converges. Therefore, recessed shapes 68 are formed on the two sides of the dissecting portion 33 when seen in the vertical direction. The air supply holes 66 similar to those of the second: embodiment are arranged at the bottoms of the respective recessed shapes 68. The dissecting operation by a dissection apparatus 22 of the embodiment is the same as that by the dissection apparatus 22 of the second embodiment. In the dissection apparatus 22 of the embodiment, the air supply holes 66 are formed in the bottoms of the recessed shapes 68. This reliably prevents the air supply holes 66 from being clogged by the blood vessel connective tissue 54, the main duct 52a, or the branch 52b. Also, a space is reserved in front of the openings of the air supply holes 66. Thus, air supply can be always performed smoothly.

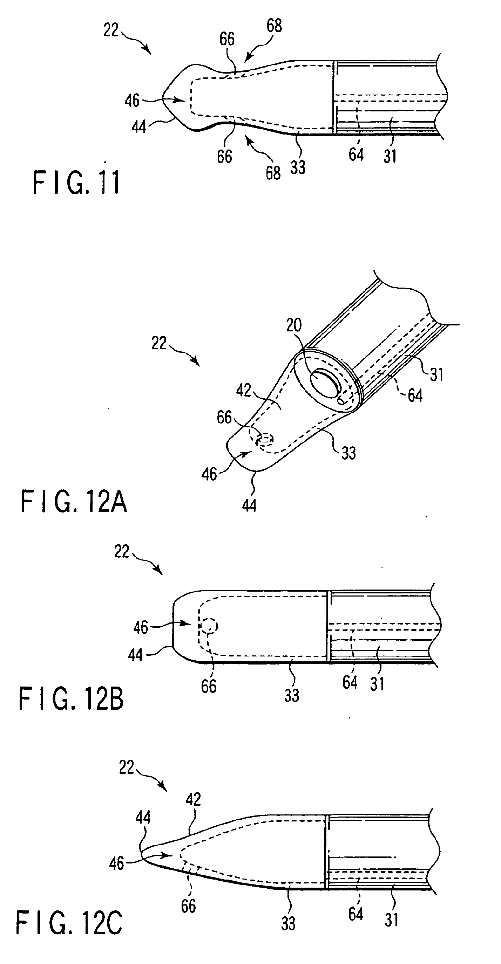

FIG. 11 shows the fourth embodiment of the present invention.

The dissecting portion 33 according to the embodiment includes almost the same shape as that of the dissecting portion 33 of the third embodiment. Note that the openings of air supply holes 66 face obliquely backward when seen in the vertical direction. When inserting the dissecting portion 33 between the blood vessel connective tissue 54 and the main duct 52a or the branch 52b, the more forward the air supply holes 66 are directed, the more the blood vessel connective tissue 54, the main duct 52a or the branch 52b tend to clog the air supply holes 66. As the air supply holes 66 of the embodiment face obliquely backward, clogging of the air supply holes 66 is prevented reliably.

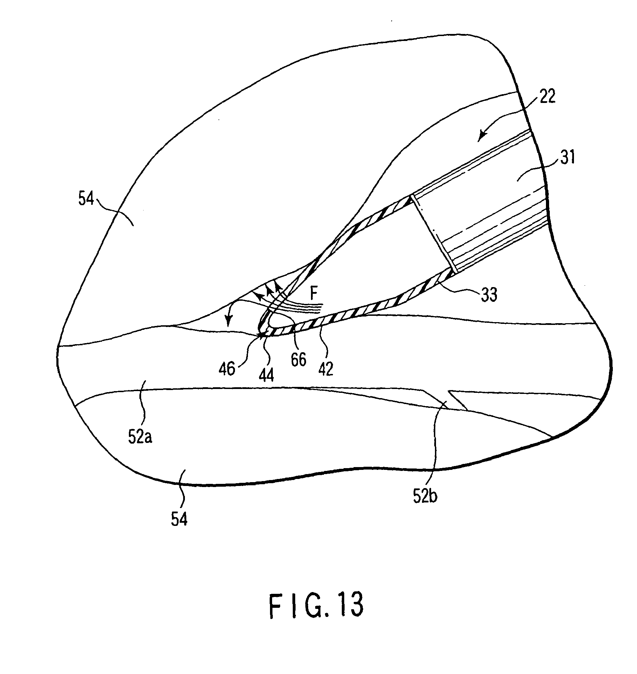

FIGS. 12A to 13 show the fifth embodiment of the present invention.

The dissecting portion 33 according to the embodiment includes the same shape as that of the dissecting portion 33 of the first embodiment. The distal end of the dissecting portion 33 includes the air supply hole 66 on the side that opposes the flat portion 42. The dissecting operation by the dissection apparatus 22 according to the embodiment is the same as that by the dissection apparatus 22 according to the second embodiment. Air supply from the air supply hole 66 enlarges the inner cavity. Air supplied from the air supply hole 66 is introduced to the space in the vicinity of the dissection target portion between the blood vessel connective tissue 54 and the main duct 52a, as indicated by arrows F. The pressure of the supplied air serves to dissect the main duct 52a from the blood vessel connective tissue 54. Hence, dissection of the main duct 52a is promoted by air supply.

## Claims

1. A dissection apparatus comprising:
a tubular main body portion (31) which extends from a proximal end side to a distal end side; and
a dissecting portion (33) which is provided to a distal end of the main body portion (31) and is adapted to dissect a blood vessel (52) from a living tissue,
wherein the dissecting portion (33) includes a flat portion (42) which is to be pressed against the blood vessel (52) and a transparent portion to observe an outside of the dissecting portion (33) from inside thereof by an endoscope (20) inserted in the main body portion (31),
wherein the transparent portion includes a mark (46) which is observable by the endoscope (20),
**characterized in that**
the mark (46) is a linear line segment which is arranged at a center of a field of view of the endoscope (20) and only partly extends through the field of view of the endoscope (20).

2. The dissection apparatus according to claim 1, **characterized in that**
the dissecting portion (33) includes a diameter decreasing portion (33) a diameter of which decreases from the proximal end side to the distal end side, and the flat portion (42) is provided to the diameter decreasing portion.

3. The dissection apparatus according to claim 1, **characterized in that**
the flat portion (42) extends from a distal end of the dissecting portion (33) to the proximal end side thereof.

4. The dissection apparatus according to claim 1, **characterized in that**
the dissecting portion (33) includes an air supply hole (66) to supply gas from inside of the dissecting portion (33) to outside thereof.

5. The dissection apparatus according to claim 4, **characterized in that**
the air supply hole (66) is adapted to be arranged within the field of view of the endoscope (20).

6. The dissection apparatus according to claim 2, **characterized in that**
the dissecting portion (33) includes an air supply hole (66) which is formed in the diameter decreasing portion (33) to supply gas from inside of the dissecting portion (33) to outside thereof.

7. The dissection apparatus according to claim 4, **characterized in that**
the air supply hole (66) is arranged on a side of the flat portion (42) in the dissecting portion (33).

8. The dissection apparatus according to claim 4, **characterized in that**
the air supply hole (66) is arranged to oppose the flat portion (42) in the dissecting portion (33).

9. The dissection apparatus according to claim 1, **characterized in that**
the mark (46) is formed of an acute-angled shape (44) of the transparent portion.

10. The dissection apparatus according to claim 1, **characterized in that**
the dissection portion (33) includes a recessed portion (68) which is arranged on a side of the flat portion (42).

11. The dissection apparatus according to claim 10, **characterized in that**
the dissection portion (33) includes an air supply hole (66) to supply gas from inside the dissection portion (33) to outside thereof, whereby the air supply hole (66) is arranged at a bottom of the recessed portion (68).

## Patentansprüche

1. Seziervorrichtung aufweisend:
ein rohrförmiges Hauptkörperteil (31), das sich von einer Proximalendseite zu einer Distalendseite erstreckt; und
ein Sezierteil (33), das an einem Distalende des Hauptkörperteils (31) bereitgestellt und dazu geeignet ist, ein Blutgefäß (52) von einem lebenden Gewebe zu sezieren,
wobei das Sezierteil (33) enthält ein flaches Teil (42) zum Drücken gegen das Blutgefäß (52) und ein transparentes Teil zum Beobachten außerhalb des Sezierteils (33) von dessen Innerem durch ein Endoskop (20), welches in das Hauptkörperteil (31) eingefügt ist,
wobei das transparente Teil eine Markierung (46) enthält, die durch das Endoskop (20) beobachtbar ist,
**dadurch gekennzeichnet, dass**
die Markierung (46) ein geradliniges Liniensegment ist, das in einem Zentrum des Sichtfelds des Endoskops (20) angeordnet ist und sich nur teilweise durch das Sichtfeld des Endoskops (20) erstreckt.

2. Seziervorrichtung nach Anspruch 1, **dadurch gekennzeichnet, dass**
das Sezierteil (33) ein Teil mit sich verringerndem Durchmesser (33) aufweist, wobei sich dessen Durchmesser von der Proximalendseite zu der Distalendseite verringert, und wobei das flache Teil (42) an dem Teil mit sich verringerndem Durchmesser bereitgestellt ist.

3. Seziervorrichtung nach Anspruch 1, **dadurch gekennzeichnet, dass**
sich das flache Teil (42) von einem Distalende des Sezierteils (33) zu dessen Proximalendseite erstreckt.

4. Seziervorrichtung nach Anspruch 1, **dadurch gekennzeichnet, dass**
das Sezierteil (33) ein Luftzuführungsloch (66) zum Führen von Gas von innerhalb des Sezierteils (33) zu dessen Äußerem enthält.

5. Seziervorrichtung nach Anspruch 4, **dadurch gekennzeichnet, dass**
das Luftzuführungsloch (66) dazu geeignet ist, in dem Sichtfeld des Endoskops (20) angeordnet zu sein.

6. Seziervorrichtung nach Anspruch 2, **dadurch gekennzeichnet, dass**
das Sezierteil (33) ein Luftzuführungsloch (66) aufweist, das in dem Teil mit sich verringerndem Diameter (33) gebildet ist, um Gas von innerhalb des Sezierteils (33) zu dessen Äußerem zu führen.

7. Seziervorrichtung nach Anspruch 4, **dadurch gekennzeichnet, dass** das Luftzuführungsloch (66) an einer Seite des flachen Teils (42) in dem Sezierteil (33) angeordnet ist.

8. Seziervorrichtung nach Anspruch 4, **dadurch gekennzeichnet, dass**
das Luftzuführungsloch (66) so angeordnet ist, dass es dem flachen Teil (42) in dem Sezierteil (33) gegenüberliegt.

9. Seziervorrichtung nach Anspruch 1, **dadurch gekennzeichnet, dass**
die Markierung (46) mit einer spitzwinkligen Form (44) des transparenten Teils gebildet ist.

10. Seziervorrichtung nach Anspruch 1, **dadurch gekennzeichnet, dass**
das Sezierteil (33) eine Aussparung (68) aufweist, die an einer Seite des flachen Teils (42) angeordnet ist.

11. Seziervorrichtung nach Anspruch 10, **dadurch gekennzeichnet, dass**
die Seziervorrichtung (33) ein Luftzuführungsloch (66) zum Führen von Gas von innerhalb des Sezierteils (33) zu dessen Äußerem enthält, wobei das Luftzuführungsloch (66) an einem unteren Ende der Aussparung (68) angeordnet ist.

## Revendications

1. Appareil de dissection comprenant :
une partie de corps principal tubulaire (31) qui s'étend d'un côté d'extrémité proximale jusqu'à un côté d'extrémité distale ; et
une partie de dissection (33) qui est prévue au niveau d'une extrémité distale de la partie de corps principal (31) et est adaptée à disséquer un vaisseau sanguin (52) à partir d'un tissu vivant,
dans lequel la partie de dissection (33) inclut une partie plate (42) qui est destinée à être pressée contre le vaisseau sanguin (52) et une partie transparente pour observer un extérieur de la partie de dissection (33) de l'intérieur de celle-ci par un endoscope (20) inséré dans la partie de corps principal (31),
dans lequel la partie transparente inclut une marque (46) qui est observable par l'endoscope (20),
**caractérisé en ce que**
la marque (46) est un segment linéaire de ligne qui est agencé au niveau d'un centre d'un champ de vision de l'endoscope (20) et ne s'étend que partiellement à travers le champ de vision de l'endoscope (20).

2. Appareil de dissection selon la revendication 1, **caractérisé en ce que**
la partie de dissection (33) inclut une partie de diamètre décroissant (33), un diamètre de laquelle décroît du côté d'extrémité proximale jusqu'au côté d'extrémité distale, et
la partie plate (42) est prévue au niveau de la partie de diamètre décroissant.

3. Appareil de dissection selon la revendication 1, **caractérisé en ce que**
la partie plate (42) s'étend d'une extrémité distale de la partie de dissection (33) jusqu'au côté d'extrémité proximale de celle-ci.

4. Appareil de dissection selon la revendication 1, **caractérisé en ce que**
la partie de dissection (33) inclut un orifice d'amenée d'air (66) pour amener un gaz de l'intérieur de la partie de dissection (33) jusqu'à l'extérieur de celle-ci.

5. Appareil de dissection selon la revendication 4, **caractérisé en ce que**
l'orifice d'amenée d'air (66) est adapté pour être agencé à l'intérieur du champ de vision de l'endoscope (20).

6. Appareil de dissection selon la revendication 2, **caractérisé en ce que**
la partie de dissection (33) inclut un orifice d'amenée d'air (66) qui est formé dans la partie de diamètre décroissant (33) pour amener un gaz de l'intérieur de la partie de dissection (33) jusqu'à l'extérieur de celle-ci.

7. Appareil de dissection selon la revendication 4, **caractérisé en ce que**
l'orifice d'amenée d'air (66) est agencé sur un côté de la partie plate (42) dans la partie de dissection (33).

8. Appareil de dissection selon la revendication 4, **caractérisé en ce que**
l'orifice d'amenée d'air (66) est agencé de façon à être en opposition à la partie plate (42) dans la partie de dissection (33).

9. Appareil de dissection selon la revendication 1, **caractérisé en ce que**
la marque (46) est formée d'une forme à angle aigu (44) de la partie transparente.

10. Appareil de dissection selon la revendication 1, **caractérisé en ce que**
la partie de dissection (33) inclut une partie en renfoncement (68) qui est agencée sur un côté de la partie plate (42).

11. Appareil de dissection selon la revendication 10, **caractérisé en ce que**
la partie de dissection (33) inclut un orifice d'amenée d'air (66) pour amener un gaz de l'intérieur de la partie de dissection (33) jusqu'à l'extérieur de celle-ci, dans lequel l'orifice d'amenée d'air (66) est agencé au niveau d'un fond de la partie en renfoncement (68).
